# EUROPÄISCHE PATENTSCHRIFT

(11) **EP 0 489 684 B1**
(45) Veröffentlichungstag und Bekanntmachung des Hinweises auf die Patenterteilung: **24.08.1994**
(21) Anmeldenummer: 91810853.1
(22) Anmeldetag: 05.11.1991
(51) Int. Cl.: A61F 2/28, A61F 2/30

(54) **Implantat zum Aufbau von Knochengewebe**
Implant for the construction of bone tissue
Implant pour la construction de tissu osseux

(30) Priorität: 05.12.1990 CH 3845/90
(43) Veröffentlichungstag der Anmeldung: 10.06.1992
(73) Patentinhaber: SULZER Medizinaltechnik AG, 8404 Winterthur (CH)
(72) Erfinder: Böhler, Nikolaus, Prof. Dr.-med., A-4020 Linz (AT); Koch, Rudolf, CH-8267 Berlingen (CH)
(74) Vertreter: Trieblnig, Adolf

(56) Entgegenhaltungen:
- EP-A- 0 307 241
- EP-A- 0 328 847
- DE-A- 2 910 627

## Beschreibung

Die Erfindung betrifft ein Implantat zum Aufbau von Knochengewebe mit einem Stützkörper aus Titan, wie es aus der EP-A-0 307 241 als bekannt hervorgeht. In der EP-A-0 328 847 ist ein Implantat zur Ergänzung eines Beckenknochens gezeigt, das zusammen mit einer künstlichen Hüftgelenkspfanne implantiert wird. Das Implantat ist für die Einbaustelle vorgeformt oder intraoperativ verformbar, wird mit Knochensplittern gefüllt und wird am Beckenknochen aufgesetzt, um das Einwachsen der Knochensplitter zu gewährleisten.

Aufgabe der Erfindung ist es, das Verfahren der Spongiosaplastik mit einem Implantat zu verbessern, das universell anwendbar ist. Diese Aufgabe wird mit der Erfindung dadurch gelöst, dass der Stützkörper ein räumliches Fachwerk bildet, dessen Balken über Knotenpunkte miteinander verbunden sind und eine Aufrauhung aufweisen, und dass Fenster, die von den Balken gebildet sind, einen Durchgang von mehr als 3 mm im Durchmesser aufweisen, um den Innenraum des Stützkörpers mit homologen oder autologen Knochengewebeteilen zu füllen.

Die Vorteile der Erfindung sind darin zu sehen, dass mit dem räumlichen Fachwerk Bausteine geschaffen werden, die sich in unterschiedlicher Anordnung am gesunden Knochen anheften und untereinander verbinden lassen, bevor sie mit Knochensplittern gefüllt und verkeilt werden. Ein weiterer Vorteil besteht darin, dass das eingesetzte Implantat von Anfang an Druckbelastungen übertragen kann, ohne dass die aufgebaute Knochenergänzung aus der beabsichtigten Form gerät.

Im folgenden wird die Erfindung anhand eines Ausführungsbeispiels im Zusammenhang mit der Zeichnung näher erläutert. Es zeigt:

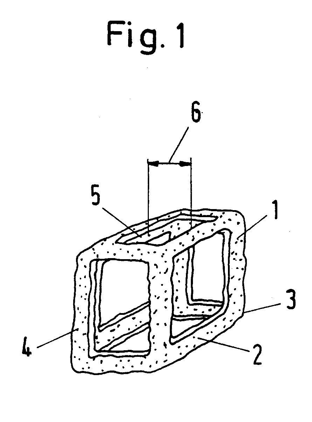

- Fig. 1: die schematische Ansicht eines als Baustein verwendbaren Stützkörpers mit seinem räumlichen Fachwerk.

In der Figur ist ein Implantat zum Aufbau von Knochengewebe gezeigt. Erfindungsgemäss bildet ein Stützkörper aus Titan ein räumliches Fachwerk, dessen Balken über Knotenpunkte miteinander verbunden sind und eine Aufrauhung aufweisen. Fenster, die von den Balken gebildet sind, weisen einen Durchgang von mehr als 3 mm im Durchmesser auf, um den Innenraum des Stützkörpers mit homologen oder autologen Knochengewebeteilen zu füllen.

In Fig. 1 ist der Stützkörper 1 als ein hohler Quader in Titan ausgeführt, dessen Kanten in Form von Balken 2 stehen geblieben sind, die in Knotenpunkten 3 zusammenlaufen. Die Stützkörper 1 werden im aufzubauenden Bereich mit Befestigungsmitteln, wie U-Nägeln und Kraupen, am gewachsenen Knochengewebe befestigt oder gegenseitig mit Draht verankert. Die Balken 2 bilden Fenster 5 zum Innenraum des Stützkörpers 1, wobei ein Durchgang 6 dieser Fenster 5 mindestens einen Durchmesser von 3 mm aufweist, um den Innenraum des Stützkörpers mit Knochengewebe zu füllen. Der Raum in und um die Stützkörper 1 wird mit Knochensplittern verkeilt. Die Oberfläche der Stützkörper 1 ist mit einer Aufrauhung 4 versehen, um das Einwachsen des Knochengewebes zu fördern. Das räumliche Fachwerk schützt mit seiner Formbeständigkeit das am ursprünglichen Knochen anwachsende Knochengewebe vor unzulässigen Belastungen.

In einer bevorzugten Ausführungsform der Erfindung hat der Stützkörper die Aussenabmessungen eines Würfels mit einer Kantenlänge zwischen 12 und 30 mm, die in einer Hauptachse um 30 bis 60 % gekürzt worden ist, während die Dicke der Balken 2 zwischen 2 und 3 mm variieren kann. Das innere Volumen ist von allen Seiten für das Stopfen mit Knochenspänen zugänglich. Jede der 6 Begrenzungsflächen kann die Berührungsfläche zum knochen für das Anwachsen der Knochenspäne bilden.

## Patentansprüche

1. Implantat zum Aufbau von Knochengewebe mit einem Stützkörper (1) aus Titan, dadurch gekennzeichnet, dass der Stützkörper (1) ein räumliches Fachwerk bildet, dessen Balken (2) über Knotenpunkte (3) miteinander verbunden sind und eine Aufrauhung (4) aufweisen, und dass Fenster (5), die von den Balken (2) gebildet sind, einen Durchgang (6) von mehr als 3 mm im Durchmesser aufweisen, um den Innenraum des Stützkörpers (1) mit homologen oder autologen Knochengewebeteilen zu füllen.

## Claims

1. An implant for the formation of osseous tissue having a support member (1) made of titanium,
**characterised in that** the support member (1) forms a three-dimensional framework, in which the beams (2) are connected to one another by points of intersection (3) and comprise a roughened surface (4), **and in that** windows (5), which are formed by the beams (2), comprise an opening of more than 3 mm in diameter, so as to fill the interior of the support member (1) with homologous or autologous parts of osseous tissue.

## Revendications

1. Implant pour la construction de tissu osseux, ledit implant comprenant un corps d'appui (1) en titane, caractérisé en ce que le corps d'appui (1) forme une armature tridimensionnelle dont les membrures (2) sont reliées entre elles par des points nodaux (3) et présentent une surface rugueuse (4), et en ce que des fenêtres (5) formées par les membrures (2) présentent un passage (6) de plus de 3 mm de diamètre pour remplir l'intérieur du corps d'appui (1) avec des éléments de tissu osseux homologues ou autologues.
